(19) Europäisches Patentamt
European Patent Office
Office européen des brevets

(11) Veröffentlichungsnummer: **0 403 718 A2**

(12) **EUROPÄISCHE PATENTANMELDUNG**

(21) Anmeldenummer: **89810958.2**

(22) Anmeldetag: **14.12.89**

(51) Int. Cl.[5]: **C08G 65/32, C08G 65/26, D06P 1/613**

(30) Priorität: **20.06.89 CH 2298/89**

(43) Veröffentlichungstag der Anmeldung: **27.12.90 Patentblatt 90/52**

(84) Benannte Vertragsstaaten: **AT BE CH DE ES FR GB IT LI NL SE**

(71) Anmelder: **CIBA-GEIGY AG**
**Klybeckstrasse 141**
**CH-4002 Basel(CH)**

(72) Erfinder: **Töpfl, Rosemarie**
**Dorneckstrasse 68**
**CH-4143 Dornach(CH)**
Erfinder: **Merz, Jürg, Dr.**
**Langgartenstrasse 51**
**CH-4105 Biel-Benken(CH)**

(54) **Styroloxid-Produkte.**

(57) Styroloxidprodukte der Formel

(1) $$R-O-\underset{Y_1}{CH}-\underset{Y_2}{CH}-O-(Alkylen-O)_m-H$$ oder

(2) $$R-O-\underset{Y_1}{CH}-\underset{Y_2}{CH}-O-(Alkylen-O)_m-\underset{Y_3}{CH}-\underset{Y_4}{CH}-OH$$

ihre sauren Ester und deren Salze, worin
R einen aliphatischen Rest mit 1 bis 24 Kohlenstoffatomen,
"Alkylen" einen Alkylenrest von 2 oder 3 Kohlenstoffatomen,
von $Y_1$ und $Y_2$ eines Phenyl und das andere Wasserstoff,
m eine Zahl von 1 bis 100 und
von $Y_3$ und $Y_4$ eines Phenyl und das andere Wasserstoff bedeuten.

Diese Produkte stellen eine neue Klasse von nichtionogenen oder anionischen Tensiden, welche als Textilveredlungsmittel, insbesondere als Emulgiermittel, Färbereihilfsmittel, Netzmittel, Entlüftungsmittel oder Foulardierhilfsmittel verwendet werden.

EP 0 403 718 A2

## Styroloxid-Produkte

Die vorliegende Erfindung betrifft neue Styroloxidprodukte sowie ihre Verwendung als Textilveredlungsmittel, insbesondere als Emulgiermittel, Färbereihilfsmittel und besonders als Netzmittel oder Foulardierhilfsmittel.

Die erfindungsgemässen Styroloxidprodukte sind dadurch gekennzeichnet, dass sie der Formel

$$(1)\qquad R{-}O{-}\underset{Y_1}{CH}{-}\underset{Y_2}{CH}{-}O{-}(Alkylen{-}O)_m{-}H \qquad \text{oder der Formel}$$

$$(2)\qquad R{-}O{-}\underset{Y_1}{CH}{-}\underset{Y_2}{CH}{-}O{-}(Alkylen{-}O)_m{-}\underset{Y_3}{CH}{-}\underset{Y_4}{CH}{-}OH$$

entsprechen und auch in Form ihrer sauren Ester und deren Salze dargestellt werden.

In den Formeln (1) und (2) bedeutet R einen aliphatischen Rest mit 1 bis 24 Kohlenstoffatomen.

"Alkylen" steht für einen Alkylenrest von 2 oder 3 Kohlenstoffatomen wie Ethylen oder Propylen oder deren Kombinationen. m bedeutet eine Zahl von 1 bis 100, vorzugsweise 2 bis 50.

Von den Substituentenpaaren ($Y_1$ und $Y_2$) und ($Y_3$ und $Y_4$) bedeutet ein Y Phenyl und das andere Y Wasserstoff.

Der Substituent R stellt vorteilhafterweise den Kohlenwasserstoffrest eines ungesättigten oder gesättigten aliphatischen Monoalkohols mit 1 bis 24 Kohlenstoffatomen dar. Der Kohlenwasserstoffrest kann geradkettig oder verzweigt sein. Vorzugsweise bedeutet R einen Alkyl- oder Alkenylrest mit 4 bis 22 Kohlenstoffatomen.

Als aliphatische gesättigte Monoalkohole können z.B. Methanol, Ethanol, Propanol, Isopropanol, Butanole, Pentanole, Hexanole, Heptanole, Octanole, Nonanole, Decanole, Laurylalkohol, Myristylalkohol, Cetylalkohol, Stearylalkohol, Arachidylalkohol oder Behenylalkohol, sowie auch Oxo-Alkohole wie insbesondere 2-Ethylbutanol, 2-Methylpentanol, 5-Methylheptan-3-ol, 2-Ethyl-hexanol, 1,1,3,3-Tetramethylbutanol, Octan-2-ol, Isononylalkohol, 3,5,5-Trimethylhexanol, Trimethyl-nonylalkohol, $C_9$-$C_{11}$-Oxoalkohol, Tridecylalkohol, Isotridecanol oder lineare primäre Alkohole (Alfole) mit 8 bis 18 Kohlenstoffatomen in Betracht kommen. Einige Vertreter der Alfole sind Alfol (8-10), Alfol (9-11), Alfol (10-14), Alfol (12-13) oder Alfol (16-18), ("Alfol" ist ein eingetragenes Warenzeichen).

Ungesättigte aliphatische Monoalkohole sind beispielsweise Vinylalkohol, Allylalkohol, Dodecenylalkohol, Hexadecenylalkohol oder Oleylalkohol.

Die Alkoholreste können einzeln oder in Form von Gemischen von zwei oder mehreren Komponenten vorhanden sein, wie z.B. Mischungen von Alkyl-und/oder Alkenylgruppen, die sich von Soja-Fettsäuren, Palmkernfettsäuren oder Talg-Oelen ableiten.

$(Alkylen{-}O)_m$-Ketten sind bevorzugt vom Ethylenglykol-, Propylenethylenglykol- oder Ethylenpropylenglykol-Typus; besonders bevorzugt ist ersterer.

m ist bevorzugt 2 bis 25.

Die Styroloxidprodukte der Formel (1) werden hergestellt, indem man zuerst den aliphatischen Monoalkohol mit 1 Mol Styroloxid verethert und dann 1 bis 100 Mol Alkylenoxid (Ethylenoxid und/oder Propylenoxid) an das erhaltene durch R-O- substituierte Phenylethanol anlagert.

Die Styroloxidprodukte der Formel (2) werden hergestellt, indem man die Produkte der Formel (1) endständig mit einem weiteren Mol Styroloxid verethert.

Die Veretherung des Monoalkohols mit Styroloxid sowie auch die endständige Styroloxidanlagerung zur Herstellung der Produkte der Formel (2) erfolgt zweckmässigerweise in einem praktisch wasserfreien oder wasserarmen Medium bei einer Temperatur von 40 bis 90 °C mit oder ohne Druck und in Gegenwart eines sauren Katalysators, wie z.B. $BF_3$-Etherat, p-Toluolsulfosäure oder vor allem konzentrierte Schwefelsäure (90-98 %). Die Säure wird dann durch Neutralisieren und Filtrieren entfernt.

Die Anlagerung des Alkylenoxids an das substituierte Phenylethanol wird nach an sich bekannten Methoden durchgeführt, wobei Ethylenoxid oder Propylenoxid oder abwechslungsweise Ethylenoxid und Propylenoxid oder Mischungen von Ethylenoxid und Propylenoxid verwendet werden.

Die sauren Ester können je nach dem Säurerest in Form von Monoester, Diester oder Halbester und als freie Säuren oder vorzugsweise als Salze z.B. Alkalimetall- oder Erdalkalimetallsalze oder Ammoniumsalze vorliegen. Als Alkalimetallsalze seien insbesondere die Natrium-, Kalium-oder Lithiumsalze, als Erdalkalimetallsalze die Magnesium- und Calciumsalze und als Ammoniumsalze die Ammonium-, Dimethylammonium-,

Trimethylammonium-, Monoethanolammonium-, Diethanolammonium- und Triethanolammoniumsalze genannt. Vorzugsweise werden die sauren Ester als Ammoniumsalze hergestellt. Mono- oder Diethanolammoniumsalze können noch weiter mit 1 bis 4 Oxyethylen-Einheiten verethert sein.

Die sauren Ester werden hergestellt, indem man das erfindungsgemässe nichtionogene Styroloxidprodukt der Formel (1) oder (2) mit einer mindestens zweibasischen Sauerstoffsäure umsetzt und gewünschtenfalls den erhaltenen sauren Ester in die obengenannten Salze überführt.

Als mehrbasische Sauerstoffsäuren für die Bildung der sauren Ester können gegebenenfalls sulfonierte, organische, vorzugsweise aliphatische Dicarbonsäuren von 3 bis 6 Kohlenstoffatomen, wie z.B. Maleinsäure, Malonsäure, Bernsteinsäure oder Sulfobernsteinsäure, oder mehrbasische anorganische Sauerstoffsäuren, wie z.B. Schwefelsäure oder Orthophosphorsäure dienen. Anstelle der Säuren können deren funktionelle Derivate wie Säureanhydride, Säurehalogenide oder Säureamide verwendet werden. Als Beispiele dieser funktionellen Derivate seien Maleinsäureanhydrid, Phosphorpentoxid, Chlorsulfonsäure und Sulfaminsäure genannt. Die Phosphorsäureester entstehen zweckmässigerweise als Gemische eines Mono- und Diesters.

Die Veresterung wird in der Regel durch einfaches Vermischen der Reaktionspartner unter Erwärmen, zweckmässig auf eine Temperatur zwischen 50° und 100° C, durchgeführt. Die zunächst entstehenden, freien Säuren können anschliessend in die entsprechenden Alkalimetall- oder Ammoniumsalze übergeführt werden. Die Ueberführung in die Salze erfolgt auf übliche Weise durch Zugabe von Basen, wie z.B. Ammoniak, Monoethanolamin, Triethanolamin oder Alkalimetallhydroxyde, z.B. Natrium- oder Kaliumhydroxyd. Gemäss einer besonders bevorzugten Ausführungsart werden saure Schwefelsäureester in Form ihrer Ammoniumsalze direkt hergestellt, indem man die nichtionogenen Styroloxidprodukte, zweckmässig in Gegenwart von Harnstoff, mit Sulfaminsäure erwärmt.

Praktisch wichtige Styroloxidprodukte entsprechen der Formel

$$(3) \qquad R_1-O-\underset{Y_1}{\underset{|}{CH}}-\underset{Y_2}{\underset{|}{CH}}-O-(CH_2CH_2O)_{m_1}-H$$

worin

$R_1$ Alkyl oder Alkenyl je mit 1 bis 22 Kohlenstoffatomen, vorzugsweise 4 bis 22 Kohlenstoffatomen von $Y_1$ und $Y_2$ eines Phenyl und das andere Wasserstoff und

$m_1$ 2 bis 40 bedeuten.

Bevorzugte mit einer anorganischen oder organischen Säure hergestellte saure Ester entsprechen der Formel

$$(4) \qquad R_1-O-\underset{Y_1}{\underset{|}{CH}}-\underset{Y_2}{\underset{|}{CH}}-O-(CH_2CH_2O)_{m_1}-X$$

oder der Formel

$$(5) \qquad R_1-O-\underset{Y_1}{\underset{|}{CH}}-\underset{Y_2}{\underset{|}{CH}}-O-(CH_2CH_2O)_{n_1}-(\underset{Z_1}{\underset{|}{CH}}-\underset{Z_2}{\underset{|}{CH}}-O)_{n_2}-X$$

oder der Formel

$$(6) \qquad R_1-O-\underset{Y_1}{\underset{|}{CH}}-\underset{Y_2}{\underset{|}{CH}}-O-(\underset{Z_1}{\underset{|}{CH}}-\underset{Z_2}{\underset{|}{CH}}-O)_{n_1}-(CH_2CH_2O)_{n_2}-X$$

worin $R_1$, $Y_1$, $Y_2$ und $m_1$ die angegebene Bedeutung haben,

von $Z_1$ und $Z_2$ eines Methyl und das andere Wasserstoff,

X den Maleinsäure-, Sulfobernsteinsäure-, Schwefelsäure- oder Phosphorsäurerest und die Summe von $n_1$ + $n_2$ 2 bis 30, vorzugsweise 4 bis 18 bedeuten.

Bevorzugte saure Ester der Formeln (4), (5) und (6) enthalten entweder eine Maleinsäureestergruppe oder eine Schwefelsäureestergruppe, die vorzugsweise in Form ihrer Alkalimetallsalze oder Ammoniumsalze vorliegen.

Von besonderem Interesse sind die sauren Phosphorsäureester der Anlagerungsprodukte der Formel (3), wobei die Phosphorsäureester zweckmässigerweise als Gemische der entsprechenden Salze eines Mono- und Diesters vorliegen.

Gewünschtenfalls können die Styroloxidprodukte der Formel (3) endständig mit einem weiteren Mol Styroloxid verethert und nötigenfalls anschliessend mit einer mindestens zweibasischen Sauerstoffsäure in den sauren Ester und in das entsprechende Salz umgewandelt werden. Sowohl die endständige Styroloxidanlagerung als auch die anschliessende Veresterung können nach den obengenannten Bedingungen durchgeführt werden.

Erfindungsgemässe Styroloxidprodukte eignen sich für die verschiedensten Zwecke in der Textilapplikation, wie z.B. Vorbehandlung, Färben oder Ausrüstung. Nichtionogene unveresterte Produkte finden insbesondere Verwendung als Hilfsmittel beim Färben von polyamidhaltigen Fasermaterialien mit anionischen Farbstoffen oder Farbstoffgemischen. Die entsprechenden sauren Ester, besonders die Dicarbonsäure-Halbester, Schwefelsäureester und in erster Linie die Phosphorsäureester hingegen werden vorallem als Netzmittel, Entlüftungsmittel und Schaumdämpfungsmittel für wässrige Systeme, insbesondere beim Färben von natürlichem oder synthetischem Fasermaterial und in erster Linie beim Färben von Cellulose-Textilmaterialien, Polyesterfasern oder natürlichen oder synthetischen Polyamidfasermaterialien verwendet. Sowohl als nichtionogene Produkte als auch als saure Ester verbessern erfindungsgemässe Styroloxidprodukte in den beschriebenen Formulierungen das Aufziehvermögen der Farbstoffe und beschleunigen dadurch die Diffusion der Farbstoffe in den Fasern.

Gegenstand der vorliegenden Erfindung ist demnach auch ein Verfahren zum Veredeln von natürlichem oder synthetischem Fasermaterial mit oder ohne entsprechende Farbstoffe, wobei die Veredelung in Gegenwart der erfindungsgemässen nichtionogenen und/oder anionischen Styroloxidprodukte durchgeführt wird.

Die Einsatzmengen, in denen die erfindungsgemässen Styroloxidprodukte den Veredelungsflotten, wie z.B. Färbeflotten oder Vor- oder Nachbehandlungsflotten zugesetzt werden, bewegen sich, je nach Substrat, zweckmässigerweise zwischen 0,5 bis 20 g, vorzugsweise 1 bis 10 g, pro Liter Flotte.

Vorteilhafte Anwendungsformen der erfindungsgemässen Styroloxidprodukte bestehen darin, dass diese als nichtionogene und/oder anionische Verbindungen mit oder ohne Wasser in Kombination mit nichtionogenen oder anionischen Dispergatoren, Fettalkoholen, Fettsäureamiden, Alkylenbisfettsäureamiden, Alkenyldicarbonsäurealkylester, Metallstearaten, Siliconölen, z.B. -Dialkylpolysiloxanen, oder auch Mineralölen oder Alkanolaminen in stabilen, netzwirkenden und/oder schaumdämpfenden Formulierungen eingesetzt werden. Derartige Zübereitungen zeichnen sich zudem dadurch aus, dass sie fähig sind, wässrige Systeme praktisch vollkommen zu entlüften. Es können somit sowohl in den Applikationsbädern als auch in den Substraten Lufteinschlüsse vermieden werden. Durch eine solche Entlüftung können Fleckenbildung bei Färbungen und Ausrüstungen ausgeschlossen werden.

Bevorzugt sind wässerige oder wasserfreie Zubereitungen, welche, bezogen auf die gesamte Zubereitung,

(A) 2 bis 50 Gew.-% eines sauren Esters des Styroloxidproduktes der Formel (1) oder (2),

(B) 5 bis 50 Gew.-% eines nichtionogenen Tensides, vorzugsweise eines aliphatischen Monoalkohols mit 6 bis 22 Kohlenstoffatomen oder eines Anlagerungsproduktes von 2 bis 80 Mol Ethylenoxid an 1 Mol eines aliphatischen Monoalkohols mit 6 bis 22 Kohlenstoffatomen oder eines Polyoxyethylenderivates eines Sorbitanfettsäureesters oder eines Styroloxidproduktes der Formel (1) oder (2) oder Gemische davon und mindestens eine der folgenden Komponenten:

(C) 1 bis 30 Gew.-% eines Silikonöls z.B. eines Dialkylpolysiloxans wie Dimethylpolysiloxan,

(D) 10 bis 60 Gew.-% eines Mineralöls z.B. Paraffinöl wie Shell Oil L 6189 oder Mineralöle Esso 301-312,

(E) 20 bis 45 Gew.-% eines Dialkylesters einer ethylenisch ungesättigten aliphatischen Dicarbonsäure, mit vorzugsweise 4 bis 12 Kohlenstoffatomen per Alkylrest, wie z.B. Maleinsäure-bis-2-ethylhexylester, Citracon-bis-2-ethylhexylester

(F) 10 bis 70 Gew.-% eines Diffusionsbeschleunigers, insbesondere eines aliphatischen oder aromatischen Carbonsäureesters wie Milchsäure-$C_4$-$C_{12}$-alkylester, Benzoesäure-$C_4$-$C_{12}$-alkylester, Benzoesäurephenylester oder Benzoesäurebenzylester, oder auch eines Alkylbenzols, wie Trimethylbenzol, Ethylbenzol,

(G) 0,5 bis 5 Gew.-% eines Salzes einer $C_{10}$-$C_{24}$-Fettsäure und eines mehrwertigen Metalles, wie z.B. Magnesiumdistearat, Calciumdibehenat oder Aluminiumtristearat und

(H) 0,5 bis 3 Gew.-% eines $C_1$-$C_4$-Alkylendiamids einer Fettsäure mit 10 bis 24 Kohlenstoffatomen, wie z.B. Methylen-bis-stearinsäureamid, Ethylen-bis-stearinsäureamid und Ethylen-bis-behensäureamid enthalten.

Weitere bevorzugte wässrige Zubereitungen bestehen darin, dass sie bezogen auf die gesamte Zubereitung,

(a) 15 bis 50 Gew.% eines nichtionischen Styroloxidproduktes der Formel (1) oder (2), besonders eines Styroloxidproduktes der Formel (3),

(b) 1 bis 5 Gew.% eines sauren Esters oder dessen Salzes, z.B. Alkalimetall- oder Ammoniumsalz, einer Verbindung der Formel

(7) 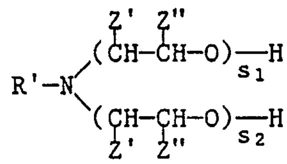

oder eines quaternierten Produktes des sauren Esters oder dessen Salzes, worin $R'$ einen Alkyl- oder Alkenylrest mit 12 bis 22 Kohlenstoffatomen, von $Z'$ und $Z''$ eines Wasserstoff, Methyl oder Phenyl und das andere Wasserstoff und $s_1$ und $s_2$ ganze Zahlen bedeuten, wobei die Summe $s_1 + s_2$ 2 bis 100 ist.

(c) 2 bis 10 Gew.% eines quaternären Ammoniumverbindung der Formel

(8) 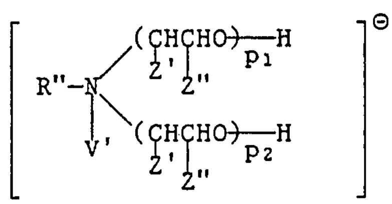 $An^{\ominus}$

in der $R''$ einen Alkyl- oder Alkenylrest mit 12 bis 22 Kohlenstoffatomen, $V'$ einen gegebenenfalls substituierten Alkylrest, wie Methyl, Ethyl, Benzyl oder $-CH_2CONH_2$, von $Z'$ und $Z''$ eines Wasserstoff, Methyl oder Phenyl und das andere Wasserstoff, $An^{\ominus}$ ein Anion einer anorganischen oder organischen Säure und $p_1$ und $p_2$ je ganze Zahlen bedeuten, wobei die Summe von $p_1$ und $p_2$ 2 bis 100 ist, und gegebenenfalls zusätzlich mindestens eine der folgenden Komponenten:

(d) 0,5 bis 25 Gew.% eines Polyalkylenglykolethers der Formel

(9) $R'''\text{-O}(\text{Alkylen-O})_q\text{H}$

worin $R'''$ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen nichtionogenen Verbindung der Formel

(10) 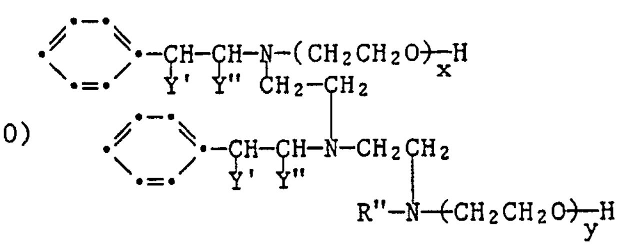

worin $R''$ einen Alkyl- oder Alkenylrest mit 12 bis 22 Kohlenstoffatomen, von $Y'$ und $Y''$ eines Phenyl und das andere Wasserstoff, und x und y ganze Zahlen bedeuten, wobei die Summe von x und y 80 bis 140 ist, enthält.

Diese Zubereitungen eignen sich insbesondere als Hilfsmittel beim Färben von natürlichen oder synthetischen Polyamidfasern, vor allem Wolle, mit anionischen Farbstoffen.

Nähere Angaben und bevorzugte Kombinationen und Anwendungsformen für Komponenten (b), (c), (d) und (e) können aus den Patentschriften DE-A-1 568 258, DE-A-1 619 385, EP-A-89004 und EP-A-312493 entnommen werden.

In den folgenden Herstellungs- und Anwendungsbeispielen beziehen sich die Prozentsätze, wenn nicht anders angegeben ist, auf das Gewicht; Teile sind Gewichtsteile. Die Mengen beziehen sich bei den

Farbstoffen auf handelsübliche d.h. coupierte Ware und bei den Hilfsmitteln auf Reinsubstanz.

Herstellungsbeispiele

Beispiel 1:

a) 540 g 3,5,5-Trimethylhexanol werden zusammen mit 15 g Schwefelsäure (96 %) auf 80 °C erwärmt. In 70 Minuten lässt man 300 g Styroloxid zutropfen, wobei die Temperatur zwischen 80 und 90 °C gehalten wird. Anschliessend wird 5 Stunden bei 86 °C nachgerührt. Das Reaktionsgemisch wird kaltgerührt und mit Natriumbicarbonatlösung neutralisiert, filtriert und die Wasserphase abgetrennt. Nachdem das überschüssige 3,5,5-Trimethylhexanol abdestilliert ist, wird im Hochvakuum fraktioniert destilliert.
Man erhält 304,6 g eines farblosen Produktes der Formel

(11) 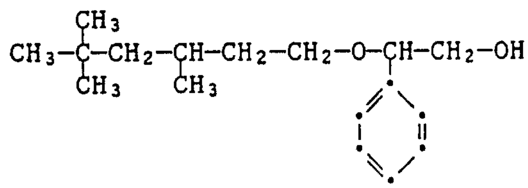

$Kp_{10}-2$: 105-106°C OH-Zahl:213

b) 263 g 3,5,5-Trimethylhexyloxy-phenylethanol der Formel (11) werden in Gegenwart von 1 % Natriummethylat mit 88 g Ethylenoxid bei 140 °C und 8 bar Druck umgesetzt.
Man erhält ein Produkt der Formel

(12) 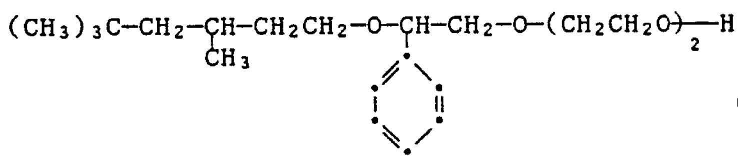

OH-Zahl: 156

c) 70,2 g des Produktes der Formel (12) werden auf 60 °C erwärmt und mit 20,8 g Harnstoff 15 Minuten gerührt. Dann gibt man 20,8 g Sulfaminsäure zu, erwärmt auf 70 °C, rührt 1 Stunde bei 70 °C, erwärmt auf 95 °C und rührt weitere 2 Stunden bei 95 °C.
Nach Zugabe von 111,8 g Wasser erhält man eine viskose, gelartige Lösung des Produktes der Formel

(13) 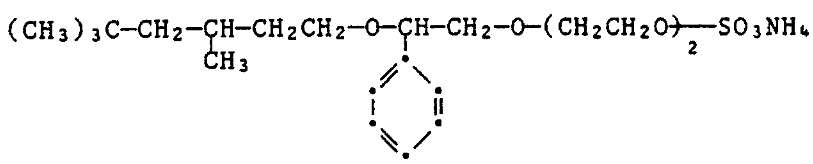

Beispiel 2:

a) 333 g n-Butanol werden zusammen mit 13,4 Schwefelsäure (96 %) auf 65 °C erwärmt. In 30 Minuten lässt man 345,6 g Styroloxid zutropfen. Dabei steigt die Temperatur auf 108 °C. Anschliessend rührt man noch 2 Stunden bei 75 °C nach. Das Reaktionsgemisch wird kaltgerührt und mit Natriumbicarbonatlösung neutralisiert und filtriert. Das überschüssige Butanol wird abdestilliert und das Produkt im Hochvakuum fraktioniert destilliert. Man erhält 201 g n-Butyloxy-phenylethanol der Formel

(14) 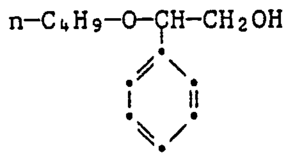

$Kp_{10^{-2}}$: 96-100°C OH-Zahl: 286

b) 192 g n-Butyloxy-phenylethanol werden in Gegenwart von 1 % Natriummethylat mit 81,8 g Ethylenoxid bei 140 °C und 6 bar Druck umgesetzt.
Man erhält ein Produkt der Formel

(15) 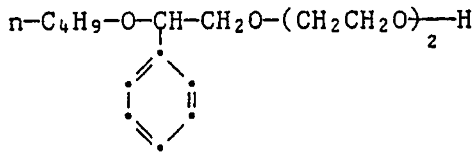

OH-Zahl: 199

c) 56,3 g des Adduktes der Formel (15) werden bei Raumtemperatur mit 9,5 g Phosphorpentoxyd unter raschem Rühren umgesetzt. Dabei steigt die Temperatur auf 65 °C. Man rührt anschliessend 4 Stunden bei Raumtemperatur. Man erhält ein Gemisch aus Mono- und Di-phosphorsäureester der Formeln

(16a) 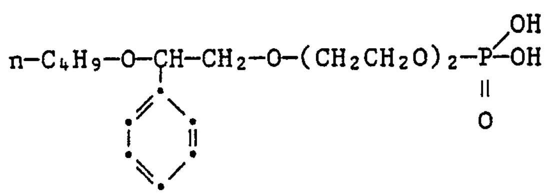

(16b) 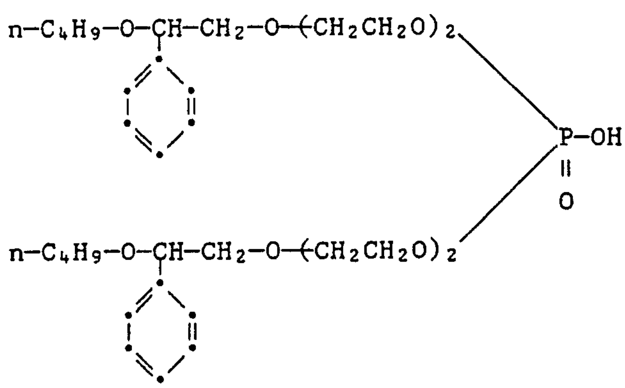

Das Reaktionsprodukt ist klar und zähflüssig.

Beispiel 3:

51 g des Adduktes der Formel

(17) 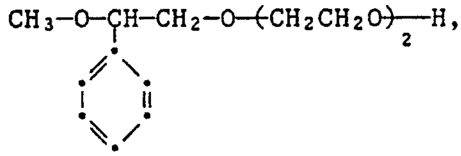

OH-Zahl: 219

hergestellt gemäss dem in Beispiel 1b) beschriebenen Verfahren, werden auf 60 °C erwärmt und mit 20,8 g Harnstoff gerührt. Hierauf gibt man 20,8 g Sulfaminsäure zu, erwärmt auf 70 °C, rührt 1 Stunde bei 70 °C, erwärmt auf 95 °C und rührt weitere 2 Stunden bei 95 °C. Nach Zugabe von 92,6 g Wasser erhält man eine mit Wasser mischbare, leicht viskose Lösung des Produktes der Formel

(18) 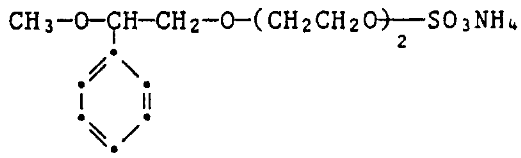

Beispiel 4:

63,75 g des Adduktes der Formel (17) werden zusammen mit 24,5 g Maleinsäureanhydrid langsam auf 70 °C erwärmt. Alsdann rührt man 1 Stunde bei 70 °C, erhöht die Temperatur auf 90 °C und rührt 3 weitere Stunden bei 90 °C. Man erhält ein hochviskoses Produkt, welches der Formel

(19) 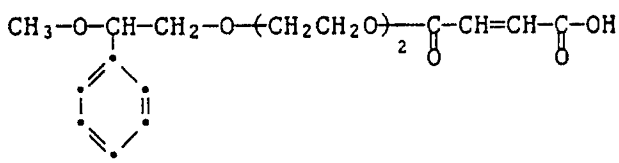

entspricht. Die Säurezahl beträgt 161.

Beispiel 5:

Zu 61,2 g des Adduktes der Formel (17) gibt man unter Rühren bei Raumtemperatur 11,35 g Phosphorpentoxid. Dabei steigt die Temperatur auf 80 °C. Man rührt anschliessend 4 Stunden bei Raumtemperatur und man erhält ein zähflüssiges Gemisch von Phosphorsäureestern der Formeln

(20a) 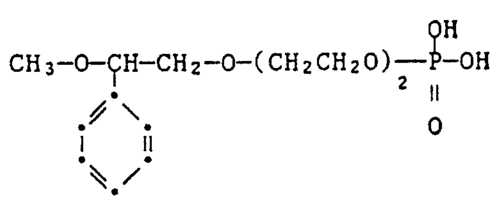

(20b) 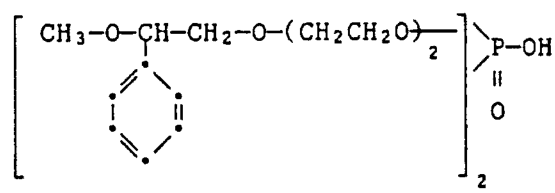

Auf ähnliche Art und Weise wie in den Beispielen 1 bis 5 beschrieben werden unter Verwendung der entsprechenden Ausgangsprodukte die folgenden nichtionogenen und anionischen Anlagerungsprodukte hergestellt:

(21)

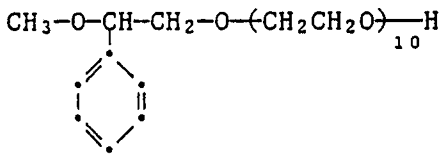

OH-Zahl: 92

(22)

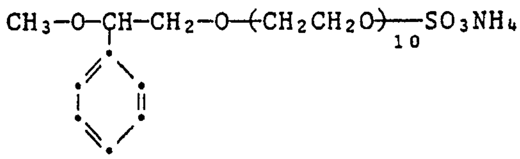

(23)

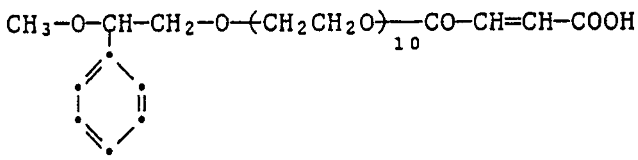

Säurezahl: 78,8

Gemisch eines Phosphorsäure-Monoesters und -Diesters der Formeln (24a) und (24b)

(24a)

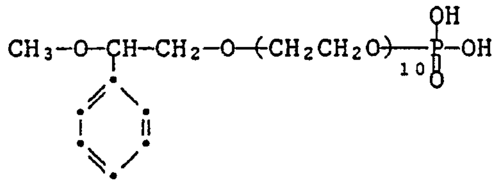

(24b)

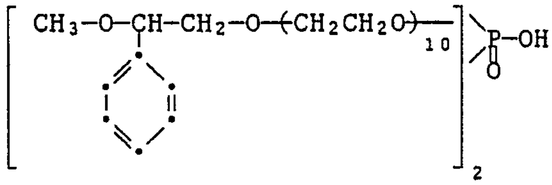

(25)

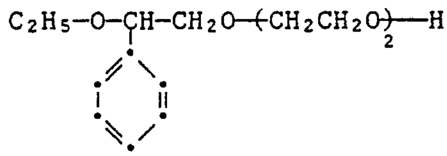

OH-Zahl: 223

(26)

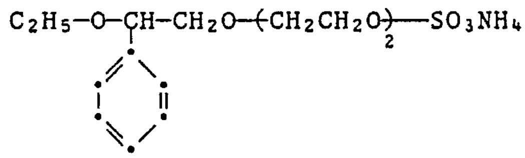

(27)

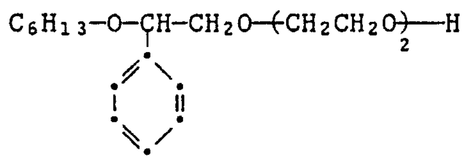

OH-Zahl: 185

(28)

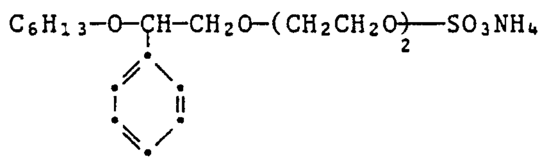

(29)

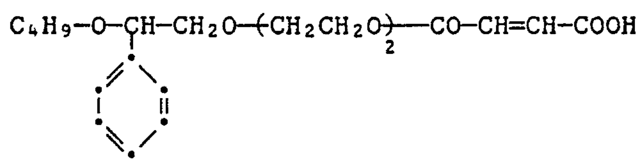

Säurezahl: 148,5

(30)

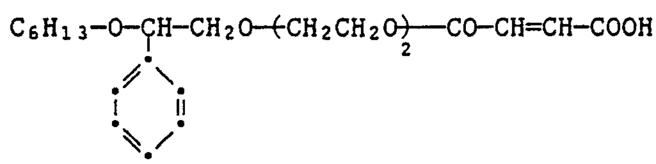

Säurezahl: 145

(31) 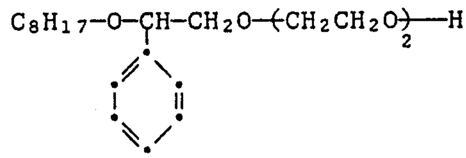

OH-Zahl: 156

(32) 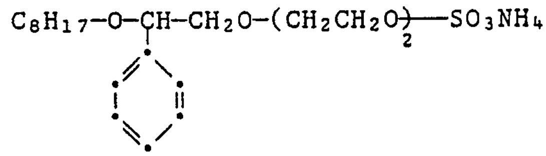

(33) 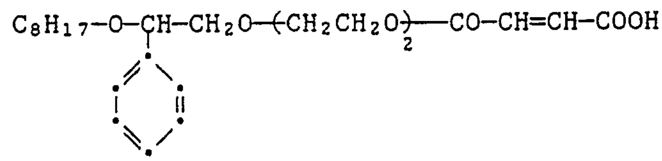

Säurezahl: 127

(34) 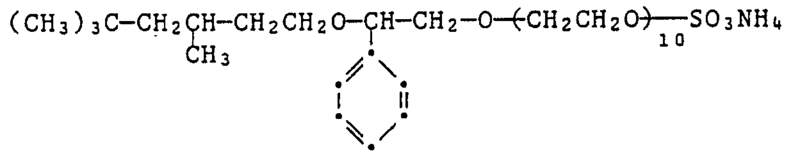

(35) 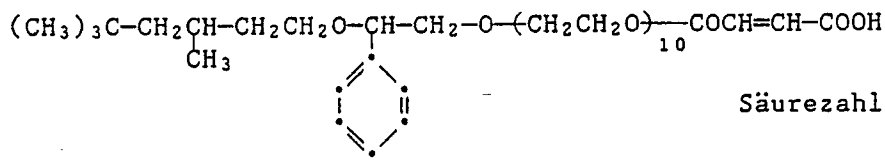

Säurezahl: 98

(36) 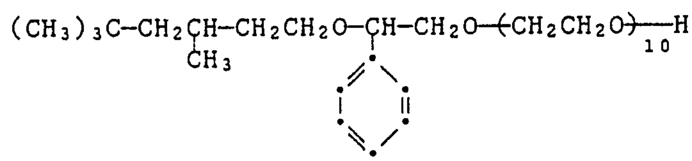

OH-Zahl: 116

Gemisch eines Phosphorsäuremonoesters oder -diesters der Formeln (37a) und (37b)

(37a) 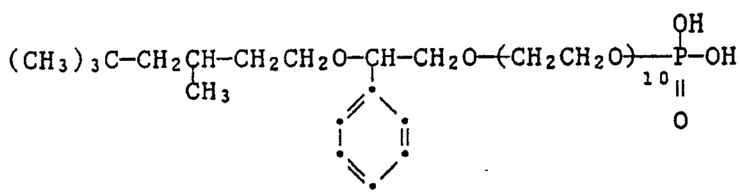

(37b) 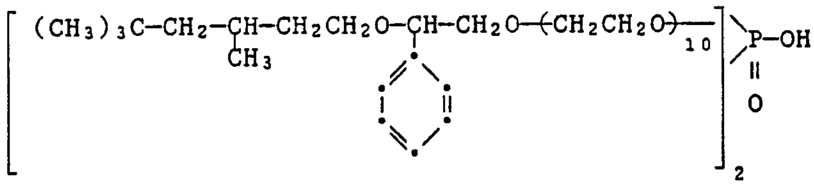

(38) 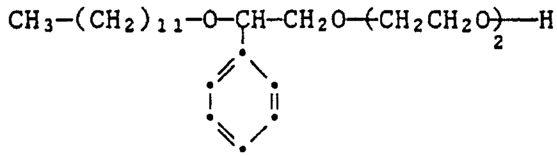

OH-Zahl: 154

(39) 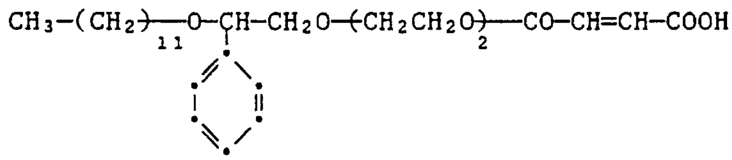

Säurezahl: 124

Gemisch eines Phosphorsäure-mono- und -diesters der Formeln (40a) und (40b)

(40a) 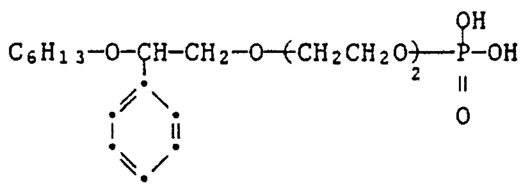

(40b) 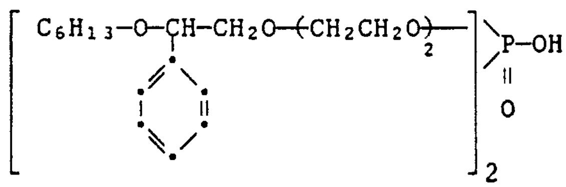

(41) 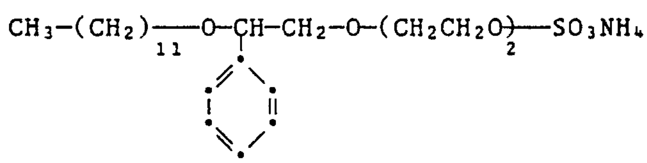

Gemisch eines Phosphorsäuremono- und -diesters der Formeln

(42) 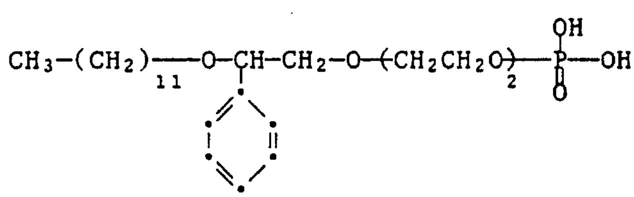

(43) 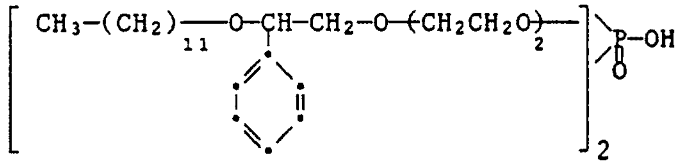

(44) 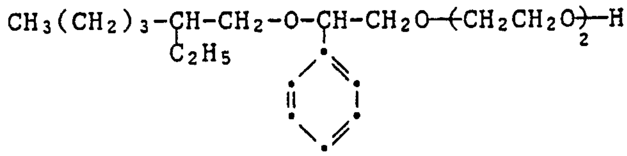

OH-Zahl: 177

(45) 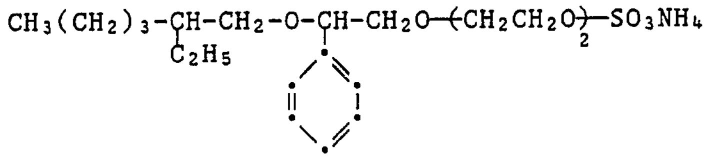

(46) 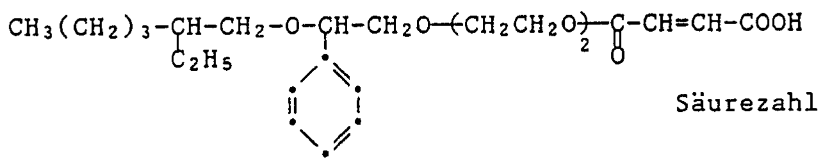

Säurezahl: 140

Gemisch eines Phosphorsäuremonoesters oder -diesters der Formeln

(47a) 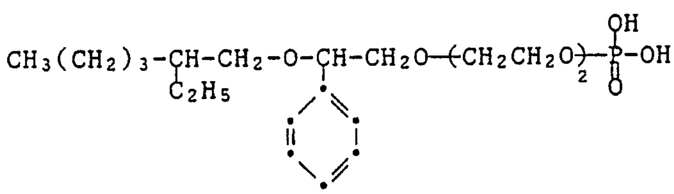

(47b) 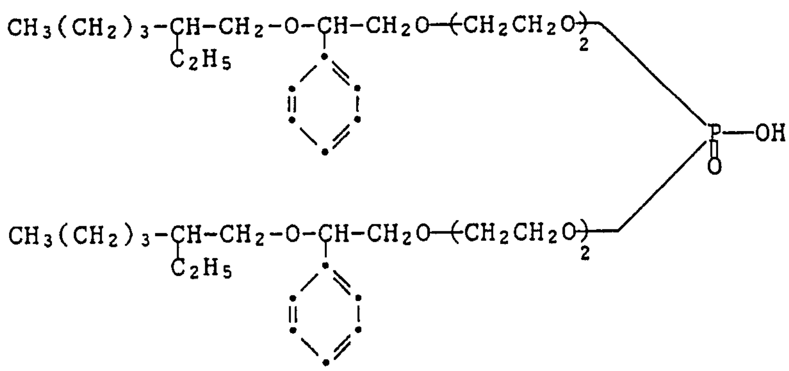

(48) 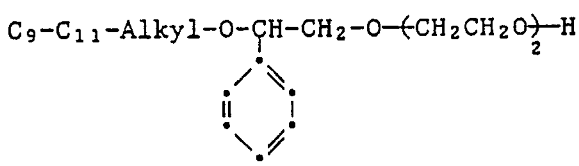

OH-Zahl: 160

(49) 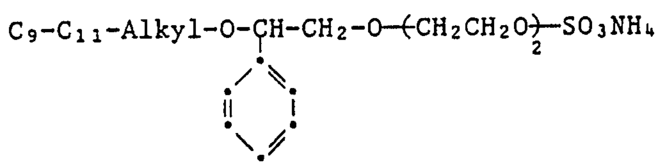

Beispiel 6:

120 g des Anlagerungsproduktes der Formel (36) (OH-Zahl: 116) werden mit 1,8 g konzentrierter Schwefelsäure (96 %) versetzt und auf 65° C erwärmt. Hierauf lässt man in 30 Minuten 30 g Styroloxid zutropfen. Die Temperatur steigt dabei auf 82° C. Anschliessend wird das Reaktionsprodukt 5 Stunden bei 75° C gerührt, mit Natriumbicarbonatlösung neutralisiert und filtriert. Das Filtrat wird eingeengt und der Rückstand getrocknet. Man erhält ein gelbes, klares Produkt der Formel

(50) 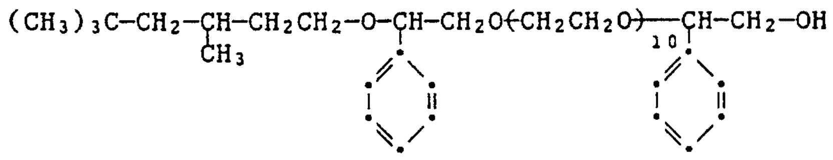

OH-Zahl: 95

Anwendungsbeispiele

Beispiel 7:

100 g Wollserge werden in einem Zirkulationsapparat bei 40° C während 15 Minuten mit einer Flotte aus 1,1 Liter Wasser eingenetzt, welche folgende Zusätze enthält:
3,2 g Essigsäure 80 %
5,0 g Natriumsulfat kalz.
1 g des Styroloxidproduktes der Formel (38).

Alsdann werden 5 g des Farbstoffes Acid Black 172 C.I. 15711 zugegeben. Nach 10 Minuten wird die Färbeflotte im Verlaufe von 45 Minuten auf 85° C erwärmt und das Färbegut unter ständiger Zirkulation 60 Minuten bei dieser Temperatur gehalten. Das Färbebad ist jetzt vollkommen erschöpft (Farbstoff auf der Faser). Anschliessend wird das Färbegut gespült und getrocknet. Man erhält ohne die konventionelle Erwärmung auf Kochtemperatur eine tiefe und echte schwarze Färbung der Wolle. Die Echtheiten entsprechen einer Färbung bei Kochtemperatur (98° C).

Aehnliche gute Ergebnisse werden erzielt, wenn anstelle des Styroloxidproduktes der Formel (38) die gleiche Menge des Bisstyroloxidproduktes der Formel (50) verwendet wird.

Beispiel 8:

In einem Zirkulationsfärbeapparat werden 100 kg Kreuzspulen aus Wolle mit einem Materialträger eingefahren. Im Ansatzbehälter werden 1200 l Wasser auf 60° C erwärmt und darin 1200 g eines wässrigen Präparates gelöst, welches bezogen auf das Präparat,
12 % Silikonöl z.B. Dimethylpolysiloxan
15 % 2-Ethylhexanol
15 % Paraffinöl
2 % eines wasserlöslichen, oberflächenaktiven Siloxanoxyalkylencopolymerisates
8 % des anionischen Styroloxidproduktes der Formel (39) und
2 % des nichtionogenen Styroloxidproduktes der Formel (38)
enthält und mit Monoethanolamin auf pH 8 eingestellt ist. Dann wird die Flotte vom Ansatzbehälter durch das Material in den Färbeapparat gepumpt und anschliessend wechselseitig zirkuliert. Durch den Zusatz des Präparates tritt eine spontane Entlüftung des Färbesystems und damit eine gute Durchdringung des Färbegutes ein.

Hierauf gibt man der Flotte 2 kg des Farbstoffes der Formel

(101) 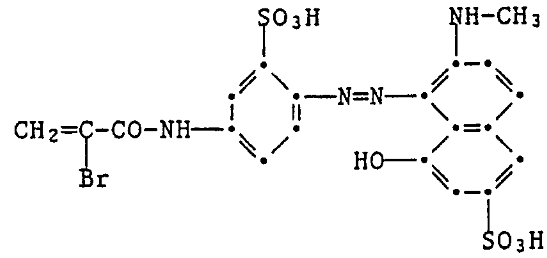

1 kg eines 1:1 Gemisches aus dem mit Chloracetamid quaternierten Anlagerungsprodukt von 7 Mol Ethylenoxid an 1 Mol eines $C_{16}$-$C_{18}$-Fettamingemisches und dem Ammoniumsalz des sauren Schwefelsäureesters des Anlagerungsproduktes von 7 Mol Ethylenoxid an 1 Mol eines $C_{16}$-$C_{18}$-Fettamingemisches (50%ige wässrige Zubereitung), erhitzt die Färbeflotte innerhalb 30 Minuten zum Sieden und färbt die Wolle 60 Minuten bei Siedetemperatur. Während der Färbung tritt praktisch keine Schaumentwicklung auf. Man erhält eine farbstarke und gleichmässige Durchfärbung der Kreuzspule.

Das in diesem Beispiel verwendete Präparat wird wie folgt hergestellt.

12 Teile Silikonöl werden in 15 Teilen 2-Ethyl-hexanol gelöst. Hierauf werden unter ständigem Rühren 15 Teile Paraffinöl, 2 Teile eines wasserlöslichen, oberflächenaktiven Siloxanoxyalkylencopolymerisates, 8 Teile des Maleinsäurehalbesters der Formel (39), 2 Teile des Styroloxidproduktes der Formel (38), 47,6 Teile Wasser und 0,4 Teile Monoethanolamin zugesetzt und 30 Minuten weitergerührt. Man erhält eine lagerstabile Formulierung mit netzender, entlüftender und besonders schaumdämpfender Wirkung.

Beispiel 9:

In einer Haspelkufe werden 100 kg Baumwollwirkware in 3000 l Wasser unter Zusatz von 3 kg eines wässrigen Präparates enthaltend, bezogen auf das Präparat,
7 % eines Silikonöls
11 % 2-Ethyl-hexanol und
11 % des anionischen Styroloxidproduktes der Formel (41) genetzt.
Innerhalb von 30 Sekunden ist die Baumwolle vollständig genetzt und entlüftet. Hierauf werden der Flotte 2 kg eines Farbstoffes der Formel

(102) 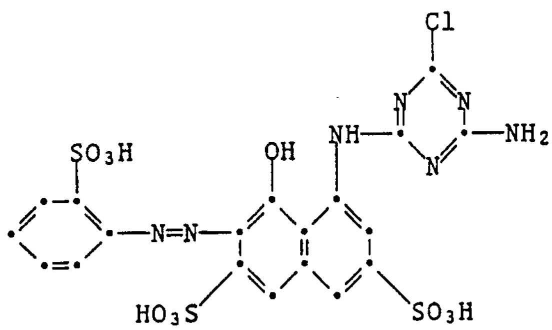

und die üblichen Chemikalien wie Elektrolyte und Alkalien zugesetzt, worauf die Ware zwei Stunden bei Siedetemperatur der Flotte gefärbt wird. Es tritt kein störendes Schäumen auf. Die gefärbte Ware ist gleichmässig und fleckenfrei durchgefärbt.

Das im Beispiel verwendete Präparat wird wie folgt hergestellt.

70 Teile Silikonöl werden zunächst in 110 Teilen 2-Ethyl-hexanol bei Raumtemperatur gelöst. Alsdann werden 110 Teile des Styroloxidproduktes der Formel (41) und nach 30 Minuten 710 Teile Wasser zugegeben, worauf es noch 30 Minuten gerührt wird. Man erhält ein lagerstabiles Produkt mit ausgezeichneten netz- und entlüftenden Eigenschaften.

Beispiel 10:

Auf einem Kurzflotten-Jet werden 100 kg Baumwolltricot in 600 Liter Wasser bei 40°C eingenetzt. In die Flotte gibt man 36 kg Natriumchlorid, 5 kg des Farbstoffes der Formel (102) sowie 0,5 kg einer Zubereitung, enthaltend

186,75 g Mineralöl, (z.B. Shell Oil L 6189),
185 g Maleinsäure-bis-2-ethylhexylester,
10 g Magnesium-distearat
8,25 g N,N-Ethylenbisstearamid
55 g eines Polyoxyethylenderivates von Sorbitantristearat mit 20 Oxyethyleneinheiten z.B. Tween 65
55 g des anionischen Styroloxidproduktes der Formel (13)

Man färbt die Ware während 45 Minuten bei 40°C. Dann erfolgt der Zusatz von 0,6 kg kalziniertem Natriumcarbonat und nach weiteren 5 Minuten von 1,2 kg einer wässrigen 36 %igen Natriumhydroxydlösung. Hierauf wird das Tricot während weiteren 40 Minuten gefärbt, anschliessend gespült und nachgewaschen. Man erhält eine echte, egale Rotfärbung des Tricots. Während des Färbeprozesses tritt kein Schäumen und keine Störung des Warenalufes auf.

Die in diesem Beispiel verwendete Zubereitung wird wie folgt hergestellt.

186,75 g Mineralöl, 185 g Maleinsäure-bis-2-ethylhexylester, 10 g Magnesiumdistearat und 8,25 g N,N-Ethylen-bis-stearamid werden unter ständigem Rühren auf 110°C erwärmt bis eine klare Lösung eintritt. Man kühlt während 5 Minuten auf 45°C ab und verteilt in der Lösung 55 g eines Polyoxyethylenderivates von Sorbitantristearat mit 20 Oxyethyleneinheiten z.B. Tween 65 und 55 g des anionischen Styroloxidproduktes der For mel (13). Man erhält eine stabile Formulierung, die insbesondere als Schaum-dämpfungsmittel in alkalischen Flotten und bei hohen Scherkräften sehr wirksam ist.

Beispiel 11:

100 Teile eines Polyestergewebes werden in eine 60°C warme Färbeflotte gegeben, die 1300 Teile Wasser, 2 g/l Ammoniumsulfat, 2,5 Teile eines Farbstoffes der Formel

(103) 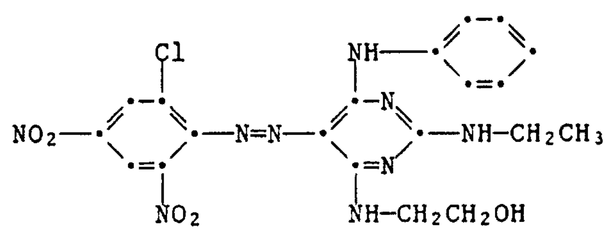

und 2 Teile einer Hilfsmittelformulierung bestehend aus

16 Teilen des anionischen Styroloxidproduktes der Formel (39)
24 Teilen des Anlagerungsproduktes von 18 Mol Ethylenoxid an 1 Mol eines $C_{12}$-$C_{18}$-Fettalkoholgemisches und
60 Teilen Benzoesäurebenzylester

enthält und mit Ameisensäure auf pH 5 gestellt ist. Man steigert die Temperatur der Flotte in Verlauf von 30 Minuten auf 130°C und färbt 60 Minuten bei dieser Temperatur.

Danach wird die Flotte auf 70°C abgekühlt, abgelassen und die Ware gespült. Man erhält ohne die übliche reduktive Nachreinigung eine egale und reibechte rote Färbung mit einer hohen Farbstoffausbeute.

Die in diesem Beispiel verwendete Hilfsmittelformulierung wird wie folgt hergestellt:

In einem heizbaren Rührgefäss werden 60 Teile Benzoesäurebenzylester auf 60°C unter ständigem Rühren erwärmt und anschliessend 24 Teile des Anlagerungsproduktes von 18 Mol Ethylenoxid an 1 Mol eines $C_{12}$-$C_{18}$-Fett alkoholgemisches und 16 Teile des Styroloxidproduktes der Formel (39). Man erhält nach Abkühlen auf Raumtemperatur eine lagerstabile Formulierung, die insbesondere beim Färben von Polyesterfasern geeignet ist.

Beispiel 12:

100 Teile eines Wickelkörpers aus texturierten Polyestergarnen werden in einen HT-Färbeapparat, welcher 800 Teile 40°C warmes Wasser, 2 Teile Ammoniumsulfat, 4 Teile eines Farbstoffes der Formel

(104) 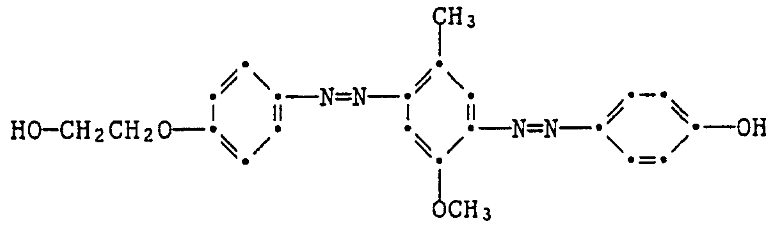

und 2 Teile der Hilfsmittelformulierung gemäss Beispiel 11 enthält und dessen Flotte mit Ameisensäure auf pH 5,5 gestellt ist, eingebracht. Danach wird die Temperatur der Flotte in Verlaufe von 40 Minuten auf 128 °C erhöht und die Ware 60 Minuten bei dieser Temperatur gefärbt. Während der Aufheizphase wird im Materialblock kein Anstieg des Differenzdruckes zwischen innen und aussen festgestellt. Anschliessend wird die Flotte auf 70 °C abgekühlt und das Substrat wie üblich reduktiv gereinigt, gespült und getrocknet. Man erhält eine farbstarke und egale orange Färbung, die sich durch gute Durchfärbung und gute Echtheiten auszeichnet.

Beispiel 13:

Ein Polyamid-(6,6)-Teppich (Schlingenflor) wird auf einem Foulard bei 25 °C mit einer Zubereitung, welche im Liter

1,2 g eines Farbstoffes der Formel

(105) 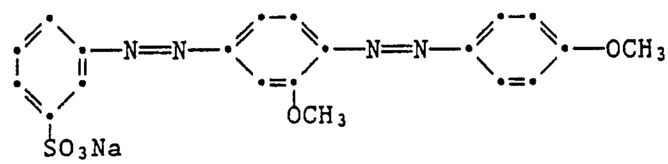

0,8 g eines Farbstoffe der Formel

(106) 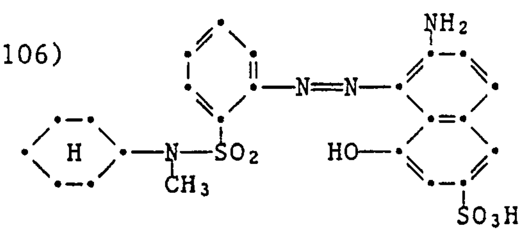

0,8 g eines Farbstoffes der Formel

(107) 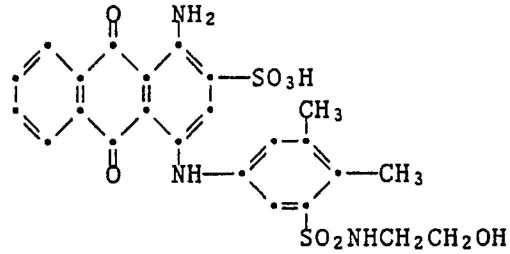

5 g des gemäss Beispiel 1 hergestellten Styroloxidproduktes der Formel (13)
2 g Verdickungsmittel und
1 g Natriumacetat

enthält und mit Essigsäure auf pH 5-5,5 eingestellt ist, imprägniert. Die Flottenaufnahme beträgt 320 %. Der imprägnierte Teppich wird mit Sattdampf im "Dog-house-steamer" bei 100 °C während 5 Minuten gedämpft. Anschliessend wird der Teppich mit kaltem Wasser gespült und getrocknet. Man erhält eine farbkräftige braune Färbung, ohne Grauschleier und mit dem erwarteten Echtheitsstandard.

Aehnlich gute Ergebnisse werden erhalten, wenn anstelle des Styroloxidproduktes der Formel (13) die gleiche Menge der anionischen Styroloxidprodukte der Formeln (32) oder (37a) mit (37b) verwendet werden.

Beispiel 14:

100 g Wollserge werden in einem Zirkulationsapparat z.B. Ahiba-Turbomat bei 40 °C während 15 Minuten mit einer Flotte aus 1,1 Liter Wasser eingenetzt, welche folgende Zusätze enthält:
3,2 g Essigsäure 80 %
5 g Natriumsulfat kalz.
2 g einer wässrigen Zubereitung enthaltend, bezogen auf diese Zubereitung, 30 Gew. % des Styroloxidproduktes der Formel (38), 10 Gew. % des Hilfsmittelgemisches $A_1$ gemäss EP-A-0089004 und 20 Gew. % Triethylenglykolmonobutylether.

Alsdann werden 4 g des Farbstoffes Acid Black 172 Cl. 15711 zugegeben. Nach 10 Minuten wird die Färbeflotte im Verlaufe von 50 Minuten auf 90 °C erwärmt und das Färbegut unter ständiger Zirkulation 60 Minuten bei dieser Temperatur gehalten. Anschliessend wird das Färbegut gespült und getrocknet.

Man erhält eine tiefe, egale und echte schwarze Färbung der Wolle. Das Färbebad ist zu 95 % erschöpft, obwohl bei 90 °C gefärbt wird. Die Echtheiten entsprechen einer konventionellen Färbung bei Kochtemperatur (98 °C).

Beispiel 15:

Vertreter der entsprechend den Beispielen 1 bis 5 hergestellten Styroloxidprodukte werden auf ihr Netzvermögen und Schaumverhalten gegenüber bekannten Produkten geprüft.

A. Netzvermögen

Nach Prüfvorschrift AL-10-1 gemäss der Untersinkmethode
Substrat: Wolldamentuch
Einsatzmenge: 1 g pro Liter Wirksubstanz

B. Schaumverhalten:

Schaumtest gemäss der Schaumstössel-Methode
Apparat: Ahiba-Texomat
Testlösung: 200 ml einer 0,1%igen wässrigen Lösung des Styroloxidproduktes
Temperatur: 25 °C
Stempeln: 60 mal
Beurteilung des Schaumvolumens: 5 Minuten nach der Schaumerzeugung

C. Oberflächenspannung

0,1 g des Styroloxidproduktes werden in 99,9 g destilliertem Wasser gelöst. Die Oberflächenspannung (dyn/cm) wird von dieser Lösung mit Hilfe eines Tensiometers bestimmt.

Die Ergebnisse in untenstehender Tabelle zeigen, dass die Styroloxidprodukte gegenüber den Vergleichsprodukten bessere Netzvermögen und Schaumverhalten aufweisen.

Tabelle

| Styroloxidprodukt der | Netzen (Untersinkzeit) Wolle | Schaumhöhe (ml) | dyn/cm (mN/m) |
|---|---|---|---|
| Formeln (40a) und (40b) | 4" | 25 | 34,3 |
| Formel (27) | 2" | 0 | 35,0 |
| Formel (31) | 7" | 0 | 33,8 |
| Formel (33) | 7" | 0 | 35,4 |
| Formel (35) | 4" | 20 | 30,4 |
| Formel (36) | 6" | 10 | 30,4 |
| Formel (12) | 8" | 0 | 30,9 |
| Ammoniumsalz des Schwefelsäureesters des Anlagerungsproduktes von 2 Mol Ethylenoxid an 1 Mol p-Nonylphenol | 8" | 225 | 30,1 |

## Ansprüche

1. Verbindungen der Formel

(1) $R-O-\underset{Y_1}{\underset{|}{C}H}-\underset{Y_2}{\underset{|}{C}H}-O-(Alkylen-O)_m-H$ oder der Formel

(2) $R-O-\underset{Y_1}{\underset{|}{C}H}-\underset{Y_2}{\underset{|}{C}H}-O-(Alkylen-O)_m-\underset{Y_3}{\underset{|}{C}H}-\underset{Y_4}{\underset{|}{C}H}-OH$

ihre sauren Ester und deren Salze, worin
R einen aliphatischen Rest mit 1 bis 24 Kohlenstoffatomen,
"Alkylen" einen Alkylenrest von 2 oder 3 Kohlenstoffatomen,
von den Substituentenpaaren ($Y_1$ und $Y_2$) und ($Y_3$ und $Y_4$) ein Y Phenyl und das andere Y Wasserstoff und
m eine Zahl von 1 bis 100 bedeuten.

2. Verbindungen gemäss Anspruch 1 dadurch gekennzeichnet, dass in Formeln (1) und (2)
R einen Alkyl- oder Alkenylrest von 4 bis 22 Kohlenstoffatomen bedeutet.

3. Verbindungen gemäss einem der Ansprüche 1 und 2 dadurch gekennzeichnet, dass in Formeln (1) und (2)
"Alkylen" Ethylen bedeutet.

4. Verbindungen gemäss einem der Ansprüche 1 bis 3, dadurch gekennzeichnet, dass in Formeln (1) und (2)
m 2 bis 25 ist.

5. Verbindungen gemäss Anspruch 1 dadurch gekennzeichnet, dass sie der Formel

(3) $R_1-O-\underset{Y_1}{\underset{|}{C}H}-\underset{Y_2}{\underset{|}{C}H}-O-(CH_2CH_2O)_{m_1}-H$

entsprechen, worin
$R_1$ Alkyl oder Alkenyl je mit 1 bis 22 Kohlenstoffatomen,
von $Y_1$ und $Y_2$ eines Phenyl und das andere Wasserstoff und
$m_1$ 2 bis 40 bedeuten.

6. Saure Ester gemäss Anspruch 1 dadurch gekennzeichnet, dass sie der Formel

$$(4) \qquad R_1-O-\underset{Y_1}{CH}-\underset{Y_2}{CH}-O-(CH_2CH_2O)_{m_1}-X$$

entsprechen, worin
$R_1$ Alkyl oder Alkenyl je mit 1 bis 22 Kohlenstoffatomen,
von $Y_1$ und $Y_2$ eines Phenyl und das andere Wasserstoff,
X den Maleinsäure-, Sulfobernsteinsäure-, Schwefelsäure- oder Phosphorsäurerest und
$m_1$ 2 bis 40 bedeuten.

7. Saure Ester gemäss Anspruch 1 dadurch gekennzeichnet, dass sie der Formel

$$(5) \qquad R_1-O-\underset{Y_1}{CH}-\underset{Y_2}{CH}-O-(CH_2CH_2O)_{n_1}-(\underset{Z_1}{CH}-\underset{Z_2}{CH}-O)_{n_2}-X$$

entsprechen, worin
$R_1$ Alkyl oder Alkenyl je mit 1 bis 22 Kohlenstoffatomen,
von $Y_1$ und $Y_2$ eines Phenyl und das andere Wasserstoff,
von $Z_1$ und $Z_2$ eines Methyl und das andere Wasserstoff,
X den Maleinsäure-, Sulfobernsteinsäure-, Schwefelsäure- oder Phosphorsäurerest und die Summe von $n_1$ + $n_2$ 2 bis 30, vorzugsweise 4 bis 18 bedeuten.

8. Saure Ester gemäss Anspruch 1 dadurch gekennzeichnet, dass sie der Formel

$$(6) \qquad R_1-O-\underset{Y_1}{CH}-\underset{Y_2}{CH}-O-(\underset{Z_1}{CH}-\underset{Z_2}{CH}-O)_{n_1}-(CH_2CH_2O)_{n_2}-X$$

entsprechen, worin
$R_1$ Alkyl oder Alkenyl je mit 1 bis 22 Kohlenstoffatomen,
von $Y_1$ und $Y_2$ eines Phenyl und das andere Wasserstoff,
von $Z_1$ und $Z_2$ eines Methyl und das andere Wasserstoff,
X den Maleinsäure-, Sulfobernsteinsäure-, Schwefelsäure- oder Phosphorsäurerest und die Summe von $n_1$ + $n_2$ 2 bis 30, vorzugsweise 4 bis 18 bedeuten.

9. Verbindungen gemäss Anspruch 1, dadurch gekennzeichnet, dass sie der Formel

$$(7) \qquad R_1-O-\underset{Y_1}{CH}-\underset{Y_2}{CH}-O-(\text{Alkylen}-O)_{m_1}-\underset{Y_3}{CH}-\underset{Y_4}{CH}-OH$$

entsprechen, ihre sauren Ester und deren Salze, worin $R_1$ Alkyl oder Alkenyl mit 1 bis 22 Kohlenstoffatomen, "Alkylen" einen Alkylenrest von 2 oder 3 Kohlenstoffatomen, von $Y_1$ und $Y_2$ eines Phenyl und das andere Wasserstoff, $m_1$ eine Zahl von 2 bis 40 und von $Y_3$ und $Y_4$ eines Phenyl und das andere Wasserstoff bedeuten.

10. Wässerige oder wasserfreie Zubereitung, welche, bezogen auf die gesamte Zubereitung,
(A) 2 bis 50 Gew.-% eines sauren Esters einer Verbindung der Formel

(1) 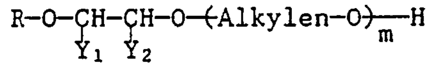 oder der Formel

(2) 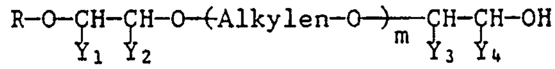

worin
R einen aliphatischen Rest mit 1 bis 24 Kohlenstoffatomen,
"Alkylen" einen Alkylenrest von 2 oder 3 Kohlenstoffatomen,
von $Y_1$ und $Y_2$ eines Phenyl und das andere Wasserstoff,
m eine Zahl von 1 bis 100, vorzugsweise 2 bis 50 und
von $Y_3$ und $Y_4$ eines Phenyl und das andere Wasserstoff bedeuten,
(B) 5 bis 50 Gew.-% eines nichtionogenen Tensides und mindestens eine der folgenden Komponenten
(C) 1 bis 30 Gew.-% eines Silikonöls,
(D) 10 bis 60 Gew.-% eines Mineralöls,
(E) 20 bis 45 Gew.-% eines Dialkylesters einer ethylenisch ungesättigten aliphatischen Dicarbonsäure,
(F) 10 bis 70 Gew.-% eines Diffusionsbeschleunigers, insbesonder eines aliphatischen oder aromatischen Carbonsäureesters oder eines Alkylbenzols,
(G) 0,5 bis 5 Gew.-% eines Salzes einer $C_{10}$-$C_{24}$-Fettsäure und eines mehrwertigen Metalls und
(H) 0,5 bis 3 Gew.-% eines $C_1$-$C_4$-Alkylendiamids einer Fettsäure mit 10 bis 24 Kohlenstoffatomen
enthält.

11. Wässerige Zubereitung, welche bezogen auf die gesamte Zubereitung
(a) 15 bis 50 Gew.% eines nichtionogenen Styroloxidproduktes der Formel

(1) 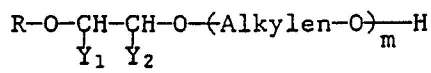 oder der Formel

(2) 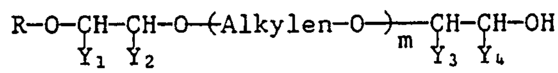

worin R einen aliphatischen Rest mit 1 bis 24 Kohlenstoffatomen,
"Alkylen" einen Alkylenrest von 2 oder 3 Kohlenstoffatomen,
von den Substituentenpaaren ($Y_1$ und $Y_2$) und ($Y_3$ und $Y_4$) ein Y Phenyl und das andere Y Wasserstoff und
m eine Zahl von 1 bis 100, vorzugsweise 2 bis 50 bedeuten,
(b) 1 bis 5 Gew.% eines sauren Esters oder dessen Salzes einer Verbindung der Formel

(7) 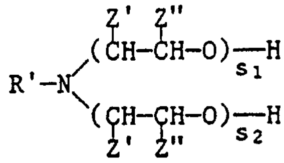

oder eines quaternierten Produktes des sauren Esters oder dessen Salzes,
worin R′ einen Alkyl- oder Alkenylrest mit 12 bis 22 Kohlenstoffatomen, von Z′ und Z″ eines Wasserstoff, Methyl oder Phenyl und das andere Wasserstoff und $s_1$ und $s_2$ ganze Zahlen bedeuen, wobei die Summe $s_1 + s_2$ 2 bis 100 ist,
(c) 2 bis 10 Gew.% einer quaternären Ammoniumverbindung der Formel

(8) 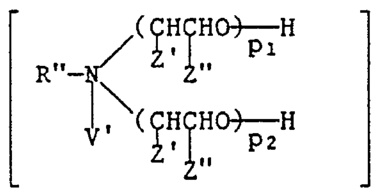 $An^{\ominus}$

in der R″ einen Alkyl- oder Alkenylrest mit 12 bis 22 Kohlenstoffatomen, V′ einen gegebenenfalls substituierten Alkylrest, von Z′ und Z″ eines Wasserstoff, Methyl oder Phenyl und das andere Wasserstoff, $An^{\ominus}$ ein Anion einer anorganischen oder organischen Säure und $p_1$ und $p_2$ ganze Zahlen bedeuten, wobei die Summe von $p_1$ und $p_2$ bis 100 ist, und gegebenenfalls zusätzlich mindestens eine der folgenden Komponenten

(d) 0,5 bis 25 Gew.% eines Polyalkylenglykolethers der Formel

(9) $R'''\text{-O}(\text{Alkylen-O})_q\text{-H}$

worin R‴ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen nichtionogenen Verbindung der Formel

(10) 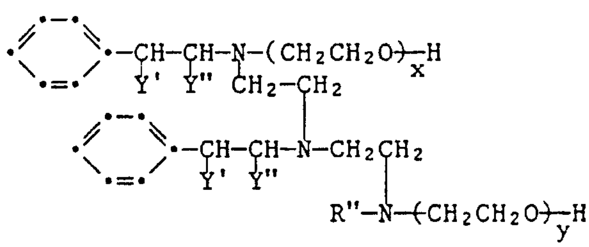

worin R″ einen Alkyl- oder Alkenylrest mit 12 bis 22 Kohlenstoffatomen, von Y′ und Y″ eines Phenyl und das andere Wasserstoff, und x und y ganze Zahlen bedeuten, wobei die Summe von x und y 80 bis 140 ist, enthält.

12. Verwendung der Verbindungen gemäss einem der Ansprüche 1 bis 9 oder der Zubereitung gemäss Anspruch 10 oder 11 beim Veredeln von natürlichem oder synthetischem Fasermaterial, insbesondere von natürlichen oder synthetischen Polyamidfasern, Polyesterfasern oder Cellulosefasern mit oder ohne entsprechenden Farbstoffen.

13. Verfahren zum Veredeln von natürlichem oder synthetischem Fasermaterial, insbesondere von natürlichen oder synthetischen Polyamidfasern, Polyesterfasern oder Cellulosefasern, mit oder ohne entsprechende Farbstoffe, dadurch gekennzeichnet, dass die Veredelung in Gegenwart von Styroloxidanlagerungsprodukten gemäss Anspruch 1 der Formel

(1) 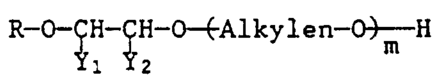 oder der Formel

(2) 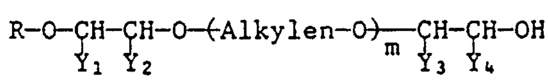

ihren sauren Estern und deren Salzen, worin

R einen aliphatischen Rest mit 1 bis 24 Kohlenstoffatomen,

"Alkylen" einen Alkylenrest von 2 oder 3 Kohlenstoffatomen,

von den Substituentenpaaren ($Y_1$ und $Y_2$) und ($Y_3$ und $Y_4$) ein Y Phenyl und das andere Y Wasserstoff und m eine Zahl von 1 bis 100 bedeuten, durchgeführt wird.